# EUROPÄISCHE PATENTSCHRIFT

(11) **EP 3 654 868 B1**
(45) Veröffentlichungstag und Bekanntmachung des Hinweises auf die Patenterteilung: **04.11.2026**
(21) Anmeldenummer: 18742450.2
(22) Anmeldetag: 13.07.2018
(51) Int. Cl.: A61B 34/30, A61B 17/00, A61B 18/20, B25J 9/06, B25J 13/08, B25J 19/00, B25J 19/02

(54) **ROBOTER ZUM BEHANDELN VON FLÄCHEN**
ROBOT FOR TREATING SURFACES
ROBOT DE TRAITEMENT DE SURFACES

(30) Priorität: 17.07.2017 DE 102017116004
(43) Veröffentlichungstag der Anmeldung: 27.05.2020
(73) Patentinhaber: KUKA Deutschland GmbH, 86165 Augsburg (DE)
(72) Erfinder: OTT, Lars-Sören, 52353 Düren (DE)
(74) Vertreter: Dorow, Florian
(86) Internationale Anmeldenummer: PCT/EP2018/069069
(87) Internationale Veröffentlichungsnummer: WO 2019/016089

(56) Entgegenhaltungen:
- EP-A1- 3 162 407
- EP-A1- 3 162 407
- WO-A1-2014/164539
- WO-A1-2014/164539
- WO-A2-2008/085758
- WO-A2-2008/085758
- DE-A1- 102015 119 424
- DE-A1- 102015 119 424
- US-A1- 2007 255 355
- US-A1- 2007 255 355
- US-A1- 2016 192 961
- US-A1- 2016 192 961

## Beschreibung

Die Erfindung bezieht sich auf einen Roboter, umfassend einen mehrere Glieder aufweisenden Arm, Antriebe zum Bewegen der Glieder, eine Steuervorrichtung zum Betätigen der Antriebe sowie einen mit dem Arm verbundenen Prozesskopf. Ferner bezieht sich die Offenbarung auf ein Verfahren zum Bearbeiten bzw. Behandeln einer eine Struktur und/oder zumindest eine Kennzeichnung aufweisenden Fläche.

Roboter, also Mehrzweckhandhabungsgeräte mit frei programmierbaren Bewegungsabläufen, werden umfassend in Industrie und Medizin eingesetzt. Dabei können Mensch und Roboter in enger Zusammenarbeit hochsensible Aufgaben lösen, wie dies durch den Roboter "LBR iiwa" der KUKA AG, Augsburg, realisiert wird. Mittels Gelenkmomentensensoren werden Kontakte sofort erkannt, so dass gefahrbringende Kollisionen ausgeschlossen sind.

Auf dem medizinischen Gebiet werden mittels Roboter bereits Operationen durchgeführt. Auch bei der Tumorbehandlung werden medizinische Roboter eingesetzt. So ist der EP 2 412 406 B1 ein medizinischer Roboter zu entnehmen, bei dem die Antriebe kraftgeregelt betrieben werden, um eine Schallquelle mit hoch intensivem fokussierten Ultraschall auf ein Lebewesen mit vorgegebener Kraft drücken zu können.

Zum Entfernen von Tattoos werden gleichfalls Roboter eingesetzt, wie der US 8 036 448 B2 zu entnehmen ist. Hautunebenheiten können jedoch beim Entfernen des Tattoos zu Problemen führen.

Die EP 3 162 407 A1 offenbart ein robotisches Behandlungssystem, welches mittels LaserStrahlung automatisiert eine unerwünschte dermatologische Veränderung, z.B. ein Tattoo auf der Haut entfernt. Dazu kann ein virtuelles Modell der zu behandelnden Hautoberfläche verwendet werden um die Laser-Bewegungen durch den Roboter zu steuern.

Aus der DE 10 2015 119 424 A1 geht ein Bearbeitungssystem eines Werkstücks hervor, welches sich mit einer Kraft- und/oder Momentengeregelten Handhabungseinrichtung entlang der Kontur eines Werkstücks bewegt und dabei Druck auf die Oberfläche des Werkstücks ausübt.

Die US 2007/0255355 A1 offenbart ein System zur Hautbehandlung durch optische Strahlung, wobei bei der Behandlung ein Über- oder Unterdruck erzeugt werden kann um Blutgefäße nahe der Hautoberfläche zu verringern, damit die Strahlung effektiver auf die zu behandelnden Hautbereiche eingebracht werden kann.

Der vorliegenden Erfindung liegt die Aufgabe zugrunde, einen Roboter zuvor beschriebener Art so weiterzubilden, dass mit hoher Präzision eine Fläche bearbeitet bzw. behandelt werden kann, um vorhandene Strukturen bzw. Kennzeichnungen zu verändern bzw. zu entfernen.

Die Aufgabe wird mit einem Roboter der eingangs genannten Art dadurch gelöst, dass die Steuervorrichtung eingerichtet ist, die Antriebe kraft- und/oder momentengeregelt zu betreiben derart, dass der Prozesskopf oder ein von dem Arm ausgehendes erstes Element eine eine Struktur und/oder zumindest eine Kennzeichnung aufweisende Fläche berührt und entlang der Fläche bei gleichzeitigem Dehnen oder gleichzeitiger Straffung der Fläche bewegt wird, dass der Prozesskopf eine Strahlung emittierende Strahlenquelle aufweist, und dass in Abhängigkeit von der Struktur und/oder der zumindest einen Kennzeichnung Wellenlänge und/oder Intensität der Strahlung beeinflussbar ist.

Man nutzt Eigenschaften z.B. eines LBR iiwa-Roboters der KUKA AG, um mittels kraft- und/oder momentengeregelter Antriebe die Glieder des Roboterarms derart zu bewegen, dass beim Kontaktieren der Fläche und beim Bewegen entlang und auf dieser die Fläche gedehnt bzw. gestrafft wird, so dass diese in dem Bereich, in dem die Strahlung auftrifft, geglättet ist. Die mit der Strahlung zu beaufschlagenden Bereiche werden somit quasi normiert, um reproduzierbare Ergebnisse zu erzielen. Gleichzeitig werden die Eigenschaften der Struktur bzw. der Kennzeichnung berücksichtigt, um in Abhängigkeit von der zu lösenden Aufgabe die Struktur bzw. Kennzeichnung zu behandeln bzw. zu bearbeiten. Möglicher Anwendungsfall ist dabei das Entfernen von Tattoos.

Ferner sieht die Erfindung auch vor, dass der Roboter einen die Fläche oder einen Bereich dieser erfassenden optischen Sensor aufweist, um mittels Bildverarbeitung den zu behandelnden bzw. zu bearbeitenden Bereich analysieren zu können und somit Wellenlänge und/oder Intensität der Strahlung zu regeln bzw. zu steuern.

Um gezielt die Temperatur der Fläche bzw. des Bereichs zu beeinflussen, ist der Roboter mit einer die Fläche kühlenden Einrichtung versehen. Dabei kann es sich um einen ein Kühlmittel abgebenden Dispenser handeln.

Das Glätten der Haut erfolgt insbesondere durch unmittelbaren Kontakt zwischen Prozesskopf und Fläche. Es besteht jedoch auch die Möglichkeit, einen Kontakt zwischen Fläche und einem Element herzustellen, der insbesondere ortsfest dem Prozesskopf zugeordnet ist. Eine insbesondere gesteuerte Relativbewegung zwischen Prozesskopf und Element ist gleichfalls möglich.

Ein Glätten kann jedoch auch alternativ oder ergänzend durch Temperatureinwirkung erzielt werden.

Aufgrund der erfindungsgemäßen Lehre ist eine automatisierte oder gegebenenfalls teilautomatisierte Bearbeitung bzw. Behandlung einer Fläche bzw. eines Bereichs dieser möglich, wobei unter Berücksichtigung der Struktur bzw. Kennzeichnung der Fläche bzw. dessen Bereichs optimale Ergebnisse erzielt werden.

In Weiterbildung der Erfindung ist vorgesehen, die Fläche bzw. den Bereich, auf die bzw. in dem die Strahlung auftrifft, zu kühlen bzw. auf Temperaturen einzustellen, um eine optimale Bearbeitung zu ermöglichen. Dabei kann die Temperaturbeeinflussung, insbesondere Kühlung, vorlaufend vor dem Bereich erfolgen, in dem die Strahlung auftrifft.

Im Falle einer Kühlung besteht die Möglichkeit, dass in Bewegungsrichtung vor dem Prozesskopf oder dem von dem Arm ausgehenden in ortsfester Beziehung zu dem Prozesskopf stehenden Element eine ein Kühlmittel abgehende Einrichtung wie Dispenser angeordnet ist.

Dadurch, dass die Bewegung des Arms des Roboters kraft- bzw. momentengeregelt erfolgt, ist sichergestellt, dass dann, wenn auf den Roboterarm zu starke Kräfte einwirken, eine weitere Bewegung unterbunden wird, so dass gefahrbringende Kollisionen mit Lebewesen ausgeschlossen sind.

Der zum Bewegen des Roboters auf der Fläche und zu deren Dehnung bzw. Straffung auf die Fläche einwirkende Druck sollte zwischen 1 KPa und 6 KPa liegen.

Ferner sollte die Fläche in dem Bereich, in dem die Strahlung auftrifft, um 0,5 % bis 2,0 % gedehnt bzw. gestrafft werden. Hierdurch ist die erforderliche Glättung erzielbar, um reproduzierbar ein Behandeln bzw. Bearbeiten durchzuführen.

Durch die erfindungsgemäße Lehre kann der Roboterarm automatisch auf die zu behandelnde bzw. zu bearbeitende Fläche abgesetzt und entlang dieser bewegt werden. Unter Zuhilfenahme insbesondere optischer Sensoren und einer Bildverarbeitung können Signale generiert werden, über die die Bewegung des Roboterarms geregelt wird.

Beim automatischen Betrieb kann durch die optische Sensorik - aber auch durch oder ergänzend durch taktile Sensorik - Lage und Positionierung der Fläche ermittelt und zur automatischen Bewegungsplanung genutzt werden. Durch Abstandssensoren bzw. taktile Sensoren kann zusätzlich die Bewegung der Fläche zu dem Roboter erfasst werden.

Es besteht auch die Möglichkeit einer Offlinebahn- bzw. Prozessplanung, so dass Prozessparameter ortsaufgelöst variiert werden können.

Die Möglichkeit eines halbautomatischen Betriebs kann auch gegeben sein. Bei dieser Maßnahme wird die Bahn des Prozesskopfes vorgegeben. Der Roboter fährt sodann auf der vorgegebenen Bahn, insbesondere mit konstanten Bearbeitungsparametern. Selbstverständlich besteht auch die Möglichkeit, in dem Roboter vorhandene Sensorik, also insbesondere Kraft- und Momentensensoren, dazu zu verwenden, sich an den zu behandelnden Bereichen bzw. einer Behandlungsbahn entlangzutasten.

Durch die optische Sensorik besteht die Möglichkeit, Form der Kennzeichnungen bzw. Strukturen sowie vorhandene Farben zu erfassen, um sodann in Abhängigkeit von den Aufgabenstellungen, wie z.B. Entfernen von Kennzeichnungen, die zu applizierende Strahlung zu variieren.

Auch besteht die Möglichkeit, auf die Fläche Markierungen aufzubringen, die für einen automatischen Bewegungsablauf genutzt werden. Entsprechende Markierungen ermöglichen ein automatisches Abfahren und eine genaue Positionierung von Bearbeitungs- bzw. Behandlungsbereichen. Denkbar ist das Aufdrucken von speziellen Marken und die Verwendung von kamerabasierten Texturerkennungsalgorithmen (Prinzip optische Maus).

Zur Strahlung wird insbesondere Laserstrahlung benutzt, wobei direkte Laserquellen oder Bearbeitungsoptiken im Zusammenspiel mit fasergeführten Lasersystemen zum Einsatz gelangen können. Auch können Lichtplätze appliziert werden, so dass man z.B. die IPL-Technik nutzt.

Bei der Verwendung von Laserquellen können durchstimmbare Laser eingesetzt werden. Auch besteht die Möglichkeit, zur Variation von Wellenlängen zwischen verschiedenen Laserquellen zu wechseln oder durch integrierte Umschaltmechanismen die gewünschte Wellenlänge aus mehreren auszuwählen.

Insbesondere ist vorgesehen, dass die Strahlung eine Wellenlänge aufweist, die im Bereich von UVB (300nm-315nm) über den UVA (315nm-400nm) sowie den gesamten sichtbaren und NIR Bereich bis hin zu einer möglichen Behandlung mit C0₂ Laserstrahlung bei einer Wellenlänge von 10640nm liegt.

Insbesondere ist vorgesehen, dass ein Laser aus der Gruppe Rubin Laser (694,3nm), Nd:YAG Laser (1064nm, frequenzverdoppelt 532nm), Erbium-YAG Laser (2940nm), Alexandrit Laser (755nm), C0₂-Laser (10640nm, 9400nm) eingesetzt wird.

Die Offenbarung zeichnet sich auch durch ein Verfahren zum Bearbeiten bzw. Behandeln einer eine Struktur und/oder zumindest eine Kennzeichnung aufweisenden Fläche mittels einer von einem Prozesskopf emittierten Strahlung, wobei der Prozesskopf von einem Arm eines Roboters ausgeht, der mehrere Glieder aufweist, die über kraft- und/oder momentengeregelte Antriebe bewegt werden, wobei der Prozesskopf oder ein diesem zugeordnetes Element auf der Fläche abgestützt wird und entlang der Fläche bei gleichzeitiger Dehnung oder Straffung dieser bewegt wird, und wobei die Intensität und/oder Wellenlänge der emittierten Strahlung in Abhängigkeit von der mittels zumindest eines Sensors erfassten Struktur und/oder der zumindest einen Markierung geregelt und/oder gesteuert wird.

Insbesondere wird in Abhängigkeit von der Struktur und/oder der zumindest einen Kennzeichnung Bewegungsbahn und Geschwindigkeit bzw. Beschleunigung des Arms geregelt bzw. gesteuert, von dem der Prozesskopf ausgeht.

Ferner kann in Abhängigkeit von der Pigmentierung der Struktur und/oder der zumindest einen Markierung und/oder der Fläche Wellenlänge und/oder Intensität der Strahlung festgelegt werden oder Auswahl eines von mehreren Prozessköpfen erfolgen.

Weitere Einzelheiten, Vorteile und Merkmale der Erfindung ergeben sich auch aus der nachfolgenden Beschreibung eines bevorzugten Ausführungsbeispiels.

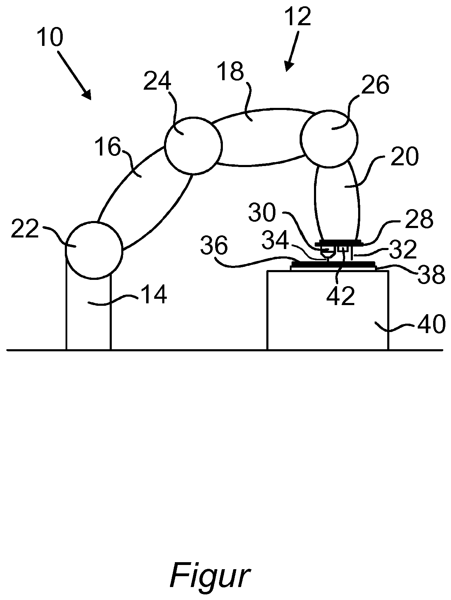

In der einzigen Figur wird ein Roboter 10 rein prinzipiell dargestellt, der als Leichtbauroboter ausgeführt werden kann. Eine konkrete Ausführungsform kann ein Roboter der KUKA AG des Typs LBR iiwa sein. Der Roboter 10 weist einen Roboterarm 12 auf, der im Ausführungsbeispiel vier Glieder 14, 16, 18, 20 aufweist. Die Glieder 14, 16, 18, 20 sind über Gelenke 22, 24, 26 miteinander verbunden, die über nicht dargestellte Antriebe bewegt werden. Bei den Antrieben handelt es sich um elektrische Antriebe, die über in dem Roboter 10 integrierte Sensoren sowie eine Steuervorrichtung kraft- und momentengeregelt bewegt werden. Eine Bewegung des Arms 12 bezüglich sechs oder mehr Freiheitsgrade ist hierdurch möglich.

Das vordere Ende des äußersten Glieds 20 weist einen Flansch 28 auf, an dem ein Prozesskopf 30 befestigt ist, in dem im Ausführungsbeispiel eine Laserstrahlquelle integriert ist.

Ferner geht vom Flansch 28 ein Abstandselement 32 aus. Mittels der von dem Prozesskopf 30 ausgehenden Laserstrahlung 34 sollen Markierungen auf eine Fläche 36 bearbeitet bzw. behandelt werden, die Oberfläche eines Körpers 38 ist, der z.B. auf einem Bearbeitungstisch 40 insbesondere ortsfest positioniert ist.

Der Körper 38 kann z.B. ein mit Leder oder Fell bezogener Gegenstand oder Leder, Fell oder Haut an sich sein, ohne dass hierdurch die erfindungsgemäße Lehre eingeschränkt wird.

In dem Arm 12, insbesondere von dem Flansch 18 ausgehend, sind des Weiteren Sensoren angeordnet, die insgesamt mit dem Bezugszeichen 42 gekennzeichnet sind. Hierbei handelt sich insbesondere um optische Sensoren, über die die Fläche 36 und damit die Struktur bzw. Kennzeichnungen der Fläche 36 erfasst werden. Die optischen Signale werden sodann von einer Bildbearbeitung bearbeitet, um in Abhängigkeit von dem Verlauf der Struktur bzw. Kennzeichnung die Bewegung des Roboterarms 12 und damit des Laserstrahls 34 regeln bzw. steuern zu können.

Dabei besteht auch die Möglichkeit, Farbe der Kennzeichnung und die der umgebenden Fläche zu erfassen, um sodann die Laserstrahlung 34 derart hinsichtlich Wellenlänge und/oder Intensität einzustellen, dass nach dem Entfernen der Kennzeichnung über die gesamte Fläche 36 eine gleiche bzw. gewünschte Pigmentierung vorliegt.

Um einen definierten Abstand zu der Fläche 36 zu erzielen, geht im Ausführungsbeispiel von dem Flansch 28 das Abstandselement 32 aus, das beim Applizieren der Laserstrahlung 34 bleibend mit der Fläche 36 in Kontakt steht und auf diese derart einwirkt, dass beim Bewegen des Arms 12 ein Dehnen der Fläche 36 erfolgt. Somit ist unabhängig von einer gegebenenfalls rauen Oberfläche eine Glättung erzielbar, die dazu führt, dass reproduzierbar Ergebnisse des Bearbeitens bzw. Behandelns erreichbar sind.

Selbstverständlich kann der Prozesskopf 30 selbst auf der Fläche 36 entlanggleiten. Entsprechend muss die Laserstrahlung 34 eingestellt werden.

Auf der Fläche 36 können auch Markierungen angebracht werden, die von den Sensoren 42 erfasst werden, um eine durch die Markierungen vorgegebene Bahnen auf der Fläche 36 abzufahren.

Die Bewegung des Arms 12 erfolgt mittels der in dem Roboter 10 integrierten Kraft- und Momentensensorik, die sicherstellt, dass zum einen die erforderliche, jedoch zulässige Kraft auf die Fläche 36 einwirkt, um das gewünschte Dehnen zu erzielen, gleichzeitig Beschädigungen nicht auftreten können. Dies ist insbesondere für den Fall von Bedeutung, dass ein Tattoo entfernt werden soll, so dass sichergestellt ist, dass gefahrbringende Krafteinwirkungen auf einen Körper unterbleiben.

Ein halbautomatischer oder automatischer Betrieb ist möglich. Bei dem halbautomatischen Betrieb fährt der Arm 12 vorgegebene Bahnen, insbesondere mit konstanten Bearbeitungsparametern ab.

Selbstverständlich besteht auch die Möglichkeit ein Bewegen durch Entlangtasten der Fläche zu erreichen. Hierzu kann ergänzend von dem Arm 12 bzw. dem Glied 20 ein taktiler Sensor ausgehen.

Beim automatischen Betrieb wird die in dem Roboterarm 12 vorhandene Sensorik bzw. mittels der Sensoren 40 die Fläche 36 bzw. der zu behandelnde Bereich erfasst, um sodann in Abhängigkeit von vorhandenen Kennzeichnungen oder Strukturen den Arm 12 so zu bewegen, dass der Laserstrahl 34 im erforderlichen Umfang gewünschte Veränderungen an der Struktur bzw. der Kennzeichnungen vornimmt.

Als Laser kommt insbesondere Rubin Laser, Nd:YAG Laser, Erbium-YAG Laser, Alexandrit Laser oder C0₂-Laser in Frage. Die Energiedichte sollte zwischen 300 mJ/cm² und 10 J/cm², insbesondere zwischen 300 mJ/cm² und 2 J/cm² liegen.

Der beim Entlanggleiten auf der Fläche 36 einwirkende Druck sollten zwischen 1 KPa und 6 KPa liegen.

Im Bereich der auftreffenden Strahlung sollte die Fläche 36 um 0,5 % bis 2,0 % gedehnt bzw. gestrafft werden.

Ist das Ausführungsbeispiel anhand des Einsatzes von Laserstrahlung beschrieben worden. In dem Prozesskopf 30 kann auch eine IPL-Technik integriert sein, so dass mit hochenergetischen Lichtblitzen eine Behandlung erfolgt.

Anstelle einer in dem Prozesskopf 30 vorhandenen Strahlenquelle kann auch ein Prozesskopf benutzt werden, der mechanische Behandlungsmittel wie Epilationseinheiten aufweist, um die Fläche 36 zu bearbeiten.

## Patentansprüche

1. Roboter (10) umfassend einen mehrere Glieder (14, 16, 18, 20) aufweisenden Arm (12), Antriebe zum Bewegen der Glieder, eine Steuervorrichtung zum Betätigen der Antriebe sowie einen mit dem Arm verbundenen Prozesskopf,
**dadurch gekennzeichnet,**
**dass** die Steuervorrichtung eingerichtet ist, die Antriebe kraft- und/oder momentengeregelt zu betreiben derart, dass der Prozesskopf (30) oder ein von dem Arm (12) ausgehendes erstes Element (32) eine eine Struktur und/oder zumindest eine Kennzeichnung aufweisende Fläche (36) berührt und entlang der Fläche bei gleichzeitigem Dehnen oder gleichzeitiger Straffung der Fläche bewegt wird, dass der Prozesskopf eine Strahlung emittierende Strahlenquelle aufweist, und dass in Abhängigkeit von der Struktur und/oder der zumindest einen Kennzeichnung Wellenlänge und/oder Intensität der Strahlung beeinflussbar ist und dass der Arm (12) eine die Fläche (36) kühlende Einrichtung aufweist.

2. Roboter nach Anspruch 1,
**dadurch gekennzeichnet,**
**dass** die Einrichtung ein Dispenser (39) ist.

3. Roboter nach zumindest einem der vorhergehenden Ansprüche,
**dadurch gekennzeichnet,**
**dass** der Arm (12) einen die Fläche (36) oder einen Bereich dieser erfassenden optischen Sensor (40) aufweist, der mit einer Bildverarbeitung verbunden ist.

4. Roboter nach zumindest einem der vorhergehenden Ansprüche,
**dadurch gekennzeichnet,**
**dass** in Abhängigkeit von Pigmentierung der Fläche (36) und/oder der Struktur bzw. der zumindest einen Kennzeichnung die Wellenlänge und/oder die Leistung der emittierten Strahlung (34) regelbar ist.

5. Roboter nach zumindest einem der vorhergehenden Ansprüche,
**dadurch gekennzeichnet,**
**dass** von dem Arm (12) ein taktiler Sensor ausgeht, mittels dessen Signale Bewegung des Arms (12) regel- und/oder steuerbar ist.

6. Roboter nach zumindest einem der vorhergehenden Ansprüche,
**dadurch gekennzeichnet,**
**dass** die Strahlenquelle zumindest ein Laser aus der Gruppe Rubin Laser (694,3nm), Nd:YAG Laser (1064nm, frequenzverdoppelt 532nm), Erbium-YAG Laser (2940nm), Alexandrit Laser (755nm), C0₂-Laser (10640nm, 9400nm) ist.

## Claims

1. Robot (10) comprising an arm (12) that comprises a plurality of links (14, 16, 18, 20), drives for moving the links, a control apparatus for actuating the drives and a process head connected to the arm,
**characterized**
**in that** the control apparatus is configured to operate the drives in a force-controlled and/or torque-controlled manner such that the process head (30) or a first element (32) extending from the arm (12) is in contact with a surface (36) comprising a structure and/or at least one identification and is moved along the surface while simultaneously stretching or tightening the surface, in that the process head comprises a radiation-emitting radiation source, and in that, depending on the structure and/or the at least one identification, the wavelength and/or intensity of the radiation can be influenced and in that the arm (12) comprises a device that cools the surface (36).

2. Robot according to Claim 1,
**characterized**
**in that** the device is a dispenser (39).

3. Robot according to at least one of the preceding claims,
**characterized**
**in that** the arm (12) comprises an optical sensor (40) that detects the surface (36) or a region thereof and that is connected to an image processing means.

4. Robot according to at least one of the preceding claims,
**characterized**
**in that** the wavelength and/or the power of the emitted radiation (34) can be controlled on the basis of the pigmentation of the surface (36) and/or the structure or the at least one identification.

5. Robot according to at least one of the preceding claims,
**characterized**
**in that** a tactile sensor whose signals enable open-loop and/or closed-loop control of the movement of the arm (12) extends from the arm (12).

6. Robot according to at least one of the preceding claims,
**characterized**
**in that** the radiation source is at least one laser from the group of ruby laser (694.3 nm), Nd:YAG laser (1064 nm; frequency-doubled: 532 nm), erbium-YAG laser (2940 nm), alexandrite laser (755 nm), CO₂ laser (10 640 nm, 9400 nm).

## Revendications

1. Robot (10) comprenant un bras (12) présentant plusieurs segments (14, 16, 18, 20), des systèmes d'entraînement pour déplacer les segments, un dispositif de commande pour actionner les systèmes d'entraînement, ainsi qu'une tête de traitement reliée au bras,
**caractérisé**
**en ce que** le dispositif de commande est conçu pour faire fonctionner les systèmes d'entraînement de manière réglée en force et/ou en couple, de telle sorte que la tête de traitement (30) ou un premier élément (32) partant du bras (12) touche une surface (36) présentant une structure et/ou au moins un marquage et soit déplacé le long de la surface en étirant ou en soumettant simultanément à une tension la surface, **en ce que** la tête de traitement présente une source de rayonnement émettant un rayonnement, **en ce que** la longueur d'onde et/ou l'intensité du rayonnement peuvent être modifiées en fonction de la structure et/ou de l'au moins un marquage, et **en ce que** le bras (12) présente un dispositif de refroidissement de la surface (36).

2. Robot selon la revendication 1,
**caractérisé**
**en ce que** le dispositif est un distributeur (39).

3. Robot selon au moins l'une des revendications précédentes,
**caractérisé**
**en ce que** le bras (12) présente un capteur optique (40) qui détecte la surface (36) ou une région de celle-ci et est relié à un système traitement d'image.

4. Robot selon au moins l'une des revendications précédentes,
**caractérisé**
**en ce que** la longueur d'onde et/ou la puissance du rayonnement émis (34) peuvent être réglées en fonction de la pigmentation de la surface (36) et/ou de la structure ou de l'au moins un marquage.

5. Robot selon au moins l'une des revendications précédentes,
**caractérisé**
**en ce qu'**un capteur tactile s'étend depuis le bras (12), au moyen des signaux duquel le déplacement du bras (12) peut être réglé et/ou commandé.

6. Robot selon au moins l'une des revendications précédentes,
**caractérisé**
**en ce que** la source de rayonnement est au moins un laser du groupe constitué par les lasers à rubis (694,3 nm), les lasers Nd:YAG (1064 nm, à fréquence doublée 532 nm), les lasers erbium-YAG (2940 nm), les lasers à alexandrite (755 nm) et les lasers à C0₂ (10640 nm, 9400 nm).
